# EUROPEAN PATENT APPLICATION

(11) **EP 2 236 607 A1**
(43) Date of publication of application: **06.10.2010**
(21) Application number: 09290250.1
(22) Date of filing: 02.04.2009
(51) Int. Cl.: C12N 15/11, A61K 48/00

(54) **Use of inhibitors of St3Gla6 activity for modulation of adipogenesis**

(71) Applicant: Sanofi-Aventis, 75013 Paris (FR)
(72) Inventor: Hall, Diana, 1007 Lausanne (CH); Jimenez, Maria, 1022 Chavannes-prés-renens (CH); Poussin, Carina, 74500 Evian-les-Bains (FR); Thorens, Bernard, 1066 Epalinges (CH)
(74) Representative: Bouvet, Philippe

(57) **Abstract**

The present invention concerns St3Gal6, a new target involved in adipogenesis modulation. Using a siRNA approach, the inventors demonstrated that decrease in St3Gal6 activity in adipose tissue induces a decrease in adipogenesis. Thus, the present invention relates to modulators of St3Gal6 activity as well as screening test for identification of modulators of the activity of this target, and their use, especially in pharmaceutical composition, to modulate adipogenesis and thus treat obesity and related disorders.

## Description

The present invention concerns St3Gal6, a new target involved in adipogenesis modulation as well as screening test for identification of modulators of the activity of this target. Further, the present invention relates to modulators of St3Gal6 activity and their use, especially in pharmaceutical composition, to modulate adipogenesis and thus to treat obesity and related disorders.

Obesity is a major risk factor for a number of disorders including hypertension, coronary artery disease, dyslipidemia, insulin resistance and type 2 diabetes. Because of the importance of the obesity epidemic, a great deal of investigation has centered on the biology of the adipocyte, including the developmental pathway by which new adipocytes are created. Adipogenesis is the process by which undifferentiated mesenchymal precursor cells become mature adipocytes. Throughout the last decade considerable progress has been made in elucidating the molecular mechanisms of adipocyte differentiation, which involve sequential activation of transcription factors from several families such as CCAAT/enhancer binding proteins (C/EBPα, α, and γ) and the nuclear hormone receptor peroxisome proliferator-activated receptor γ (PPARγ) (Rosen, E.D. et *al*., 2002). PPARγ is described as a "master regulator" of adipogenesis since it has been shown to be both sufficient and necessary for adipogenesis both *in vitro* and *in vivo.* Recently, new transcription factors have been described to participate in adipogenesis such as KLF family (KLF2, 5 and KLF15) (Banerjee, S. S. et *al*., 2003; Gray, S. M. et *al*., 2002), Ebf family (Jimenez, M. A. et al., 2007) and Krox 20 (Chen, Z. et *al.,* 2005), suggesting that the transcriptional cascade occurring during adipogenesis is much more complex than previously thought. Furthermore, signaling molecules and/or receptors such as the Wnt family of secreted proteins (Kang S. et *al*., 2007), sonic hedgehog protein, Notch receptor have also been described to be involved in molecular events leading to adipocyte formation.

These last years, an emerging concept has linked the molecular events leading to adipocyte development to the extracellular matrix (ECM) remodeling in the developing fat pad. Indeed, the developing mesenchymal cell undergoes a dramatic alteration of cell morphology from stelate-shaped to sphere. These changes in cell morphology are paralleled by dramatic changes in the levels and the types of cytoskeletal, extracellular matrix and related components such as actin, fibronectin and collagen (Grégoire F. M. *et al.,* 1998; Hausman, G.J. *et al.* 1996). Interestingly, adipose tissue contains a rich ECM, whose composition varies throughout life with changes in fat mass (Chun, T. et *al*., 2006 ; Gagnon, A. M., J. *et al*.1998; Mehlhorn, A. T., P *et al*., 2006 ; Nakajima, I. S. *et al.* 2002). The ECM not only influences the integrity of the structural system that supports cells, but also influences, via cell-surface receptors, cell-cell and cell-matrix interactions the molecular and signaling events that take place in the cells during the differentiation process. Thus, extracellular and intracellular events are coupled to regulate adipogenesis.

Storage of fat in adipose tissue is limited and exceeding this capacity leads to accumulation of lipids in others tissues, in particular in muscle, liver, and the endocrine pancreas, and to the secretion by adipocytes of various adipokines. The adipose tissue consists of several deposits located at different anatomical sites which may originate from distinct precursors and which have different physiological functions and pathophysiological roles. The visceral, as opposed to the subcutaneous adipose depots, may contribute more to the defects associated with the metabolic syndrome.

Cannabinoid 1 receptors have been identified in all organs playing a key role in glucose metabolism and type 2 diabetes, i.e. adipose tissue, the gastrointestinal tract, the liver, the skeletal muscle and the pancreas. Rimonabant, the first selective cannabinoid receptor 1 (CB1 R) antagonist in clinical use, has been shown to reduce food intake and body weight thus improving glucose metabolism regulation.

However, there is still a need for novel therapeutic targets for the treatment of obesity.

St3 β-galactosidase α2,3-sialyltransferase 6 (St3gal6) is an enzyme which belongs to the α2,3-sialyltransferase family and is present in the Golgi apparatus. St3gal6 transfers sialic acid moiety to glycolipid and /or glycoproteins (Okajima, T., S. *et al.,* 1999 ; Sewell R. *et al.,* 2006). This enzyme has been described to be involved in the translocation or targeting of proteins to the cell membrane or to any compartment in the cell. Furthermore, it has been involved in extracellular protein modifications, particularly in cancer (Oyama T., *et al*., 2006).

The inventors have now found that St3Gal6 plays a critical role in adipocytes differentiation. St3Gal6 is thus considered as a new relevant target for modulation of adipogenesis and for the treatment of obesity and related disorders. Inhibition of St3Gal6 can also be used to decrease adipogenesis for reduction of subcutaneous and visceral fat accumulation.

### Detailed description of the invention

The present invention is dawn to methods for regulating adipogenesis and metabolic function in adipocytes.

The present invention consists in the use of inhibitors of St3Gal6 activity for modulation of adipogenesis, in particular for treatment of obesity and related disorders. The invention also concerns pharmaceutical composition containing such modulators of adipogenesis and related disorders and screening test for such modulators.

Through a transcriptomic approach, the inventors identified genes whose expression was correlated with body weight gain in cohorts of C57BI/6 mice fed a high fat diet. Then, they conducted a second analysis in order to evaluate the changes in gene expression induced by rimonabant treatment of the high fat diet fed mice. Genes which have never been described before in adipocyte biology, but which might be involved in important biological processes such as signaling, modification of extracellular matrix proteins, and gene transcription were retained. These genes could be important for adipogenesis especially since they might be involved in the mechanism by which rimonabant reduces fat mass in mice. In this context, St3Gal6 was identified as involved in adipocytes metabolism, especially in new signalling pathway. More generally, this gene appears to play a role in adipogenesis and control of adipose tissue development in obesity.

The present invention consists in identification of modulators of St3Gal6 activity. Such modulators can be any compound or molecule able to modulate St3Gal6 activity in particular small molecules, lipids and siRNA.

Modulators of St3Gal6 activity can be identified by detecting the ability of an agent to modulate the activity of St3Gal6. Inhibitors of St3Gal6 are any compound able to reduce or inhibit, totally or partially, the activity of St3Gal6. Inhibitors of St3Gal6 include, but are not limited to, agents that interfere with the interaction of St3Gal6 with its natural partner in the intracellular compartment and agents that reduce St3Gal6 expression, both at transcriptional and translational levels.

For example, in order to identify a compound able to modulate the enzymatic activity of St3Gal6, the activity of sialylation can be measured in a sample containing St3Gal6 activity after addition of a candidat compound and compared with the activity of sialylation present in a control sample.

For example, whole protein extracts from adipose tissue of patients can be used to sialylate the type I disaccharides, on a preparative scale. In practice, a candidat compound can be added to a sample and the activity of sialylation can be measured by the quantification of the disappearance of the acceptor type I disaccharides seen by MNR, according to the method described in Baisch *et al,* 1998. Therefore, an inhibitor compound can be identified when a decrease in sialylation is measured compared to a control sample containing no candidat compound.

In another embodiment, the expression of St3Gal6 is modulated through RNA interference, using small interfering RNAs (siRNA) or small hairpin RNAs (shRNAs). Therefore, in one aspect, the present invention relates to double stranded nucleic acid molecules including small nucleic acid molecules, such as short interfering nucleic acid (siNA), short interfering RNA (siRNA), double-stranded RNA (dsRNA), micro-RNA (miRNA), and short hairpin RNA (shRNA) molecules able to mediate RNA interference (RNAi) against St3Gal6 gene expression, including cocktails of such small nucleic acid molecules and suitable formulations of such small nucleic acid molecules.

The phenomenon of RNAi mediated gene silencing has been described first in the Caenorhabditis elegans system, in which microinjection of long double stranded RNA molecules was reported. The mechanism of RNA mediated gene inactivation seems to be slightly different in the various organisms that have been investigated so far. However, in all systems, RNA mediated gene silencing is based on post-transcriptional degradation of the target mRNA induced by the endonuclease Argonaute2 which is part of the so called RISC complex. Sequence specificity of degradation is determined by the nucleotide sequence of the specific antisense RNA strand loaded into the RISC complex.

The introduction into cells of a siRNA compound results in cells having a reduced level of the target mRNA and, thus, of the corresponding polypeptide and, concurrently, of the corresponding enzyme activity.

siRNAs specific for St3Gal6, as described herein, can be used as modulators of St3Gai6 activity, in order to reduce the translation of St3Gal6 mRNA. More particularly, siRNA specific for St3Gal6 can be used to reduce adipogenesis and thus to treat obesity and related diseases.

In one embodiment, the invention features a double stranded nucleic acid molecule, such as a siRNA molecule, where one of the strands comprises nucleotide sequence having complementarity to a predetermined St3Gal6 nucleotide sequence in a target St3Gal6 nucleic acid molecule, or a portion thereof.

The RNA molecule can be used modified or unmodified. An example of modification is the incorporation of tricylo-DNA to allow improved serum stability of oligonucleotide.

In one embodiment, the predetermined St3Gal6 nucleotide sequence is a St3Gal6 nucleotide target sequence described herein (SEQ ID NO. 1 and SEQ ID NO. 3).

Due to the potential for sequence variability of the genome across different organisms or different subjects, selection of siRNA molecules for broad therapeutic applications likely involves the conserved regions of the gene. Thus in one embodiment, the present invention relates to siRNA molecules that target conserved regions of the genome or regions that are conserved across different targets. siRNA molecules designed to target conserved regions of various targets enable efficient inhibition of St3Gal6 gene expression in diverse patient populations.

In one embodiment, the invention features a double-stranded short interfering nucleic acid molecule that down-regulates expression of a target St3Gal6 gene or that directs cleavage of a target RNA, wherein said siRNA molecule comprises about 15 to about 28 base pairs, preferably 19 base pairs. A siRNA or RNAi inhibitor of the instant invention can be chemically synthesized, expressed from a vector or enzymatically synthesized.

In a particular embodiment, the siRNA specific for St3Gal6 are shRNA having sequence SEQ ID NO. 5 or SEQ ID NO. 6 or SEQ ID NO. 7. In a preferred embodiment, the siRNA specific for St3Gal6 are shRNA having sequence SEQ ID NO. 5. The use of a siRNA according to the present invention leads to reduction of the mRNA level from 5 % to 20 %, preferably from 5 % to 15 %, more preferably from 5 % to 10 % of the mRNA level of the corresponding wild type cell. The wild type cell is the cell prior to the introduction of the nucleic acid encoding the siRNA compound, in which the targeted mRNA is not degraded by a siRNA compound.

Inhibitors of St3Gal6 activity can be administrated by any suitable route, both locally or systemically depending on the nature of the molecule and the expected effect. SiRNA can be administrated locally in case of double strand molecule directly in the targeted tissue, or administrated through a vector in case of shRNA, according to protocols used in the art.

In one embodiment, RNAi is obtained using shRNA molecules. ShRNA constructs encode a stem-loop RNA. After introduction into cells, this stem-loop RNA is processed into a double stranded RNA compound, the sequence of which corresponds to the stem of the original RNA molecule. Such double stranded RNA can be prepared according to any method known in the art including vitro and *in vivo* methods as, but not limited to, described in Sahber *et al* (1987), Bhattacharyya *et al*, (1990) or US 5, 795, 715.

For *in vivo* administration, shRNA can be introduced into a plasmid. Plasmid-derived shRNAs present the advantage to provide the option for combination with reporter genes or selection markers, and delivery via viral or non viral vectors. The introduction of shRNA into a vector and then into cells ensure that the shRNA is continuously expressed. The vector is usually passed on to daughter cells, allowing the gene silencing to be inherited.

The present invention also provides vectors comprising the polynucleotides for expression of shRNA expression of the invention. These vectors are for example AAV vector, retroviral vector in particular lentiviral vector, adenoviral vector which can be administered by different suitable routes including intravenous route, intramuscular route, direct injection into subcutaneous tissue or other targeted tissue chosen according to usual practice.

The route of administration of siRNA varies from local, direct delivery to systemic intravenous administration. The advantage of local delivery is that the doses of siRNA required for efficacy are substantially low since the molecules are injected into or near the target tissue. Local administration also allows for focused delivery of siRNA. For such direct delivery, naked siRNA can be used. "Naked siRNA" refers to delivery of siRNA (unmodified or modified) in saline or other simple excipients such as 5% dextrose. The ease of formulation and administration of such molecules makes this an attractive therapeutic approach. Naked DNA can also be formulated into lipids especially liposomes.

Systemic application of siRNA is often less invasive and, more importantly, not limited to tissues which are sufficiently accessible from outside. For systemic delivery, siRNA can be formulated with cholesterol conjugate, liposomes or polymer-based nanoparticules. Liposomes are traditionally used in order to provide increased pharmacokinetics properties and/or decreased toxicity profiles. They allow significant and repeated success *in vivo* delivery. Currently, use of lipid-based formulations of systemic delivery of siRNA, especially to hepatocytes, appears to represent one of the most promising near-term opportunities for development of RNAi therapeutics. Formulation with polymers such as dynamic polyconjugates - for example coupled to N-acetylglucosamine for hepatocytes targeting - and cyclodextrin-based nanoparticules allow both targeted delivery and endosomal escape mechanisms. Others polymers such as atelocollagen and chitosan allow therapeutic effects on subcutaneous tumor xenografts as well as on bone metastases.

SiRNA can also be directly conjugated with a molecular entity designed to help targeted delivery. Given the nature of the siRNA duplex, the presence of the inactive or sense stand makes for an ideal site for conjugation. Examples of conjugates are lipophilic conjugates such as cholesterol, or aptamer-based conjugates.

Cationic peptides and proteins are also used to form complexes with the negatively charged phosphate backbone of the siRNA duplex.

These different delivery approaches can be used to target the St3Gal6 siRNA into the relevant tissue, especially adipose tissue. For such targeting, siRNA can be conjugated to different molecules interacting with pre-adipocytes and adipocytes, as for example ligands interacting with lipids transporters, receptors, insulin receptor or any molecule known in the art.

Another object of the invention is a pharmaceutical composition, which comprises, as active principle, a modulator of St3Gal6 according to the present invention. These pharmaceutical compositions comprise an effective dose of at least one modulator according to the invention, and at least one pharmaceutically acceptable excipient. Said excipients are chosen according to the pharmaceutical form and the administration route desired, among usual excipients known of one of skill in the art.

The invention also consists in a method for modulation of adipogenesis. Such method can be used to treat obesity or related diseases. Such method can also be used in order to decrease fat accumulation in a cosmetic purpose.

Modulators of St3Gal6 activity are useful in therapeutics to modulate adipogenesis, in particular in the treatment and prevention of obesity related disorders, in particular type 2 diabetes, dyslipidemia, elevated blood pressure, insulin resistance, cardiovascular disorders and more generally metabolic syndromes.

The present invention, according to another of its aspects, relates to a method for the treatment of the above pathologies, which comprises the *in vivo* administration to a patient of an effective dose of a modulator of St3Gal6 according to the invention.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

According to usual practice, the dosage suitable to each patient is determined by the physician according to the administration route, the weight and response of the patient.

St3Gal6 inhibitors are also useful for cosmetic applications in order to reduce disgraceful fat accumulation.

For cosmetic applications, inhibitors of St3Gal6 can be incorporated in a suitable formulation for topical use. The inhibitors of St3Gal6 can both be small molecules or siRNA as previously described.

The invention is now described by reference to the following examples, which are illustrative only, and are not intended to limit the present invention.

### Brief description of the figures

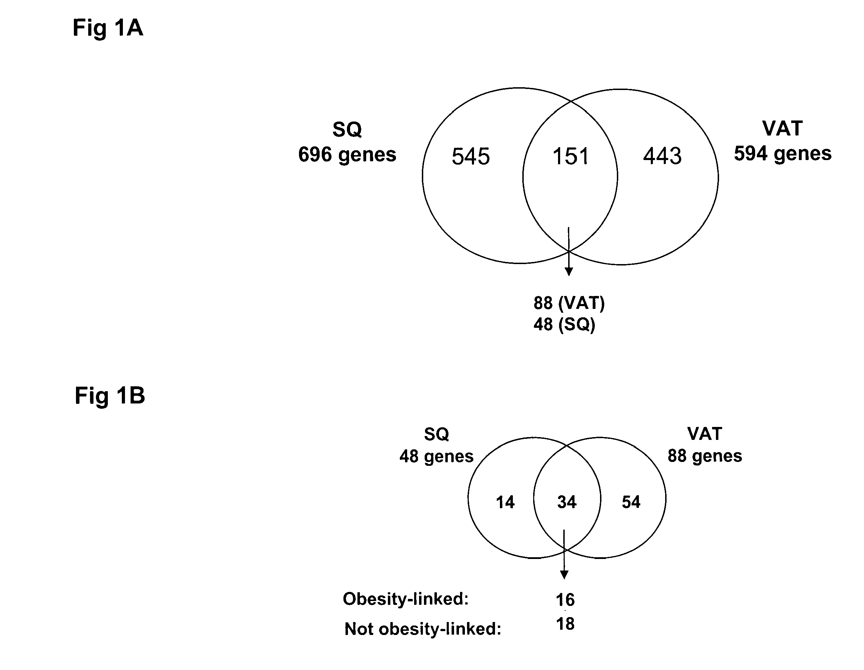

**Figure 1****: Selection of critical adipose tissue regulatory genes**. The Venn diagrams illustrate the selection of genes based on the following criteria. **1)** Similar regulation by high fat feeding in subcutaneous (SCAT or Sq) and visceral (VAT). 151 genes were selected (48 for SCAT and 88 for VAT). **2)** Among those 151 genes, selection of genes regulated by rimonabant treatment (14 for SCAT and 54 for VAT). This led to the selection of 34 genes regulated in both tissues by high fat feeding and rimonabant. Among those genes, 16 have expression level correlated with body weight of L, M and H groups (obesity-linked) and 18 are regulated by HFD to the same level in each subgroup (not obesity-linked).

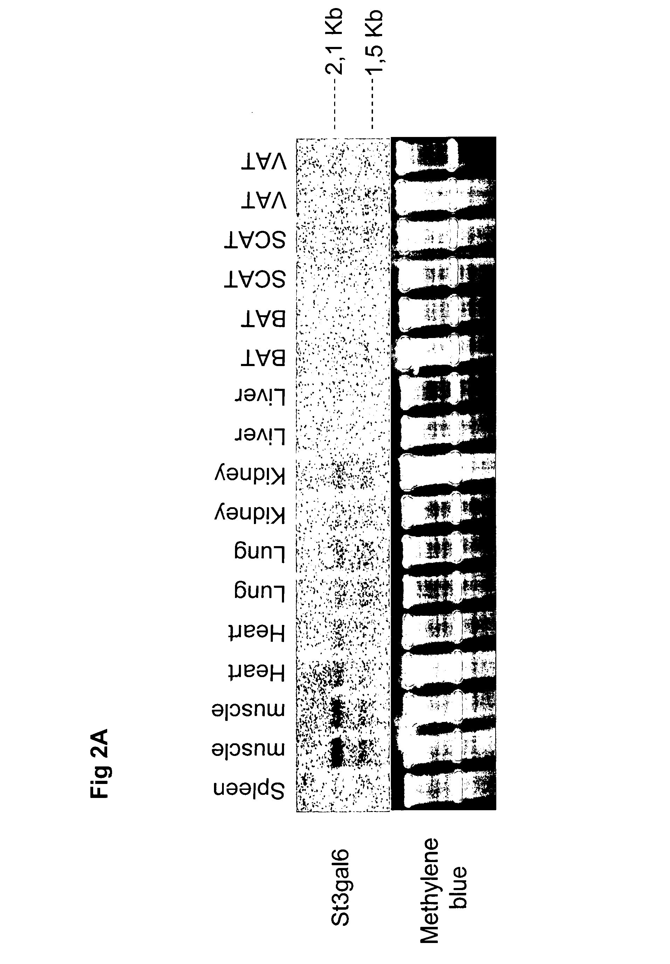

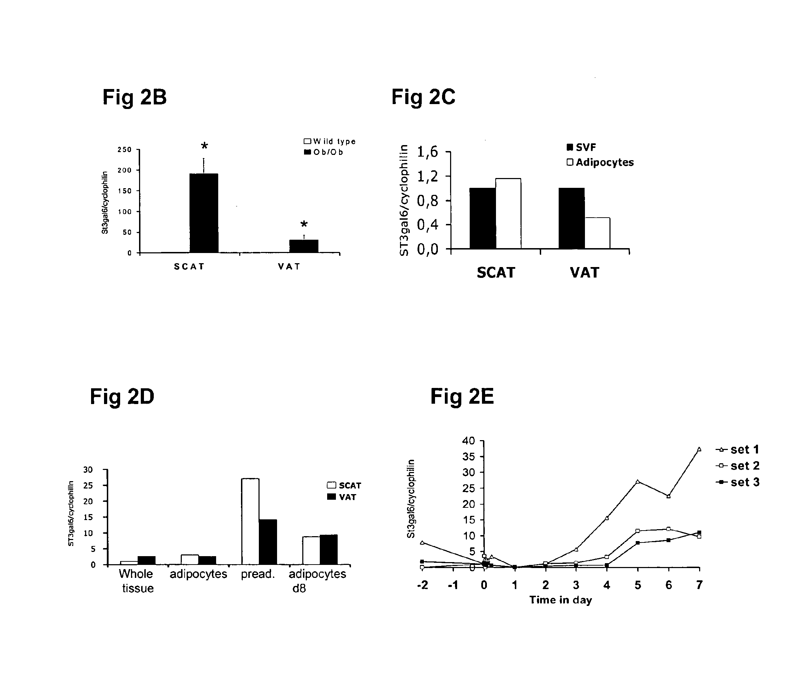

**Figure 2****: St3Gal6 expression in various tissue and cell types A)** Northern Blotting for St3gal6 showing mRNA expression in various mouse tissues: spleen, muscle (gastrocnemius), heart, lung, kidney, liver, brown adipose tissue (BAT), subcutaneous (SCAT) and visceral (VAT) adipose tissues. As a control the membrane is stained with methylene blue. The size of St3gal6 mRNA is shown on the right. **B to E:** mRNA levels of St3gal6 measured by RT-PCR **B)** in SCAT and VAT of wild-type and Ob/Ob mice (n=5) * p<0.05, data are shown as mean ± sd and expressed as fold increase relative to the control SCAT set at 1. **C)** in SVF and isolated adipocytes of mice (n=5 mice pooled for each extraction, experiment was repeated 3 times, a representative experiment is shown). Data are expressed as fold increase relative to SCAT SVF expression. **D)** in human whole tissue SCAT and VAT, isolated adipocytes, isolated preadipocytes and adipocytes differentiated *in vitro.* Data are expressed as levels relative to whole tissue SCAT expression set arbitrary at 1. **E)** in 3T3-L1 cells prior DMI treatment day-2 and after DMI treatment until day 7. N=3 sets of cells. Data are represented as levels relative to the expression at day 0.

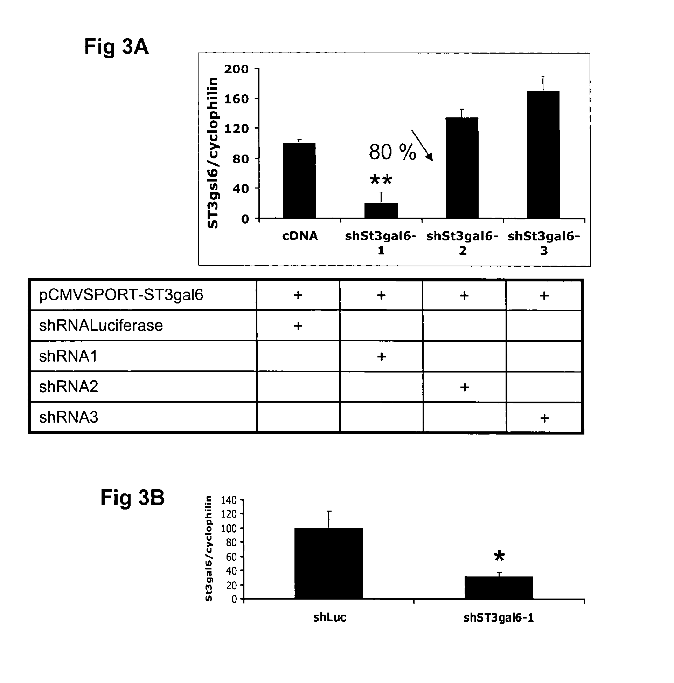

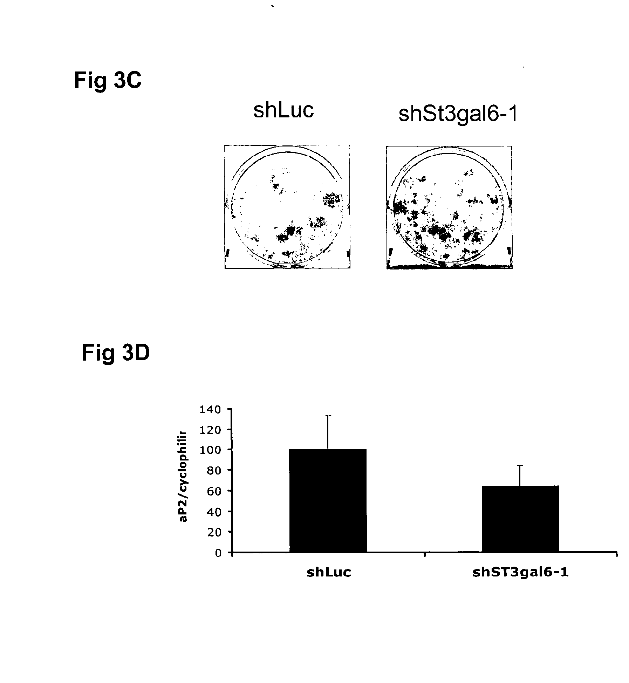

**Figure 3****: Knockdown of St3Gal6 expression and activity by shRNA. A)** shRNA transfection into 293T cells. pSIREN retroviral plasmids containing shRNA sequences against St3gal6 were co-transfected with pCMVSPORT expressing plasmid. As a control for shRNA construct, an shRNA against the firefly luciferase protein (shRNA luciferase) was used and 3 shRNA for St3gal6 were tested. **B)** 3T3-L1 cells were transduced with retroviruses containing shRNA directed against luciferase (shLuc) or St3gal6 (shSt3gal6). mRNA levels were measured by RT-PCR prior differentiation. **C)** Oil-red-O pictures of differentiated 3T3-L1 at day 9. **D)** aP2 (marker of differentiation) mRNA expression measured by RT-PCR in the same cells as in C) at day 9. Results are expressed as mean±sd *P<0.05, **P<0.01. n=3.

### Material and Methods

### Animals treatment

C57BL/6J mice, which are obesity-prone (5), were fed for 6 months with a high fat diet (HFD). After 6 months of HFD, mice exhibited scattered body weights with various degrees of glucose intolerance (measured by a glucose tolerance test. The HFD mice were separated into 3 groups displaying the same level of glucose intolerance but with low (L), medium (M) or high (H) body weights and treated them, as well as normal chow (NC) fed mice, for one month with vehicle or rimonabant (10 mg.kg⁻¹.day⁻¹), to normalize their body weight. The treatment also normalized glucose tolerance, as described previously (25).

***RNA preparation, labelling and hybridization on cDNA microarrays.*** RNA from 5 different mice per group was extracted from visceral and subcutaneous adipose tissues using peqGOLD Trifast^{™} (peqlab) and chloroform-isoamylalcool (24:1) extraction. RNA was precipitated with isopropanol and purified by passage over RNeasy columns (Qiagen). RNA quality was checked before and after amplification with a Bioanalyzer 2100 (Agilent). RNA was reverse transcribed and RNA was amplified with MessageAmp^{™} kit (Ambion). A Mouse Universal Reference (Clontech) was similarly amplified and both adipose tissue and reference RNAs were labeled by an indirect technique with Cy5 and Cy3 according to published protocols (de Fourmestraux et al., J. Biol. Chem. 2004 279:50743-53). Labeled RNAs were hybridized to microarrays containing 17664 cDNAs prepared at the DNA Array Facility of the University of Lausanne. Scanning, image, and quality control analyses were performed as previously published (de Fourmestraux et al., J. Biol. Chem. 2004 279:50743-53). Data were expressed as log₂ intensity ratios (Cy5/Cy3), normalized with a print tip locally weighted linear regression (Lowess) method and filtered based on spot quality and incomplete annotation. All analysis were performed with the R software for statistical computing available at the Comprehensive R Archive Network (cran.us.r-project.org/).

### Cell culture

3T3-L1 cells were cultured in DMEM (Gibco) with 10 % FBS (Gibco) at 5% CO₂. After retroviral infection (see below), cells were allow to grow to confluence in either 100-mm or 60-mm dishes in DMEM with 10 % FBS. Once confluence was reached, cells were exposed to differentiation medium containing dexamethasone (1µM), insulin (5 µg/ml), and isobutylmethylxanthine (0.5 µM) (DMI). After 2 days cells were maintained in medium containing insulin (5 µg/ml) until ready for harvest at 7 days.

### Oil-red-O staining

After 7 to 10 days of differentiation, cells were washed once in PBS and fixed with formaldehyde (Formalde-fresh; Fisher) for 15 minutes. The staining solution was prepared by dissolving 0.5 g oil-red-O in 100 ml of isopropanol; 60 ml of this solution was mixed with 40 ml of distilled water. After 1 hour at room temperature the staining solution was filtered and added to dishes for 4 hours. The staining solution was then removed and cells were washed twice with distilled water.

### shRNA constructs

shRNAs were constructed using the RNAi-Ready pSIREN-RetroQ ZsGreen (Clontech). Target sequences for St3Gal6 were designed by querying the Whitehead siRNA algorithm (http://jura.wi.mit.edu/bioc/siRNAext/) as well as the siRNA designer software from Clontech (http://bioinfo.clontech.com/rnaidesigner/); at least two sequences represented by both algorithms were subcloned into the pSIREN vectors (Clontech) using the EcoRI and BamH1 restriction sites. The target sequences for St3Gal6 correspond to SEQ ID NO.1 and SEQ ID NO.3. Three shRNA specific for St3Gal6 were prepared: shSt3Gal6-1 (SEQ ID NO.5), shSt3Gal6-2 (SEQ ID NO.6) and shSt3Gal6-3 (SEQ ID NO.7). As a negative control, we used the siRNA sequence against luciferase: shLuc (SEQ ID NO.8).

### Transfection of shRNA constructs

The specificity of shRNAs was tested in 293T HEK cells co-transfected using calcium-Phosphate methods described in (14) with expression vectors containing St3Gal6 cDNA and the RNAi-Ready pSIREN-RetroQ ZsGreen vector expressing either the shRNA against lucifeare (control shLUC) or St3Gal6 (shSt3Gal6). RT-PCR analysis was performed on cell RNA-extraction 24h after transfection.

### Generation of retroviral constructs and retroviral infections

Retroviruses were constructed in the RNAi-Ready pSIREN-RetroQ ZsGreen (pSIREN Clontech). Viral constructs were transfected using calcium-phosphate method described in (14) into 293 HEK packaging cells along with constructs encoding gag-pol and the VSV-G protein. Supernatants were harvested after 48h in presence of 3µm of Trichostatin A (Sigma) and either used immediately or snap frozen and stored at -80°C for later use. Viral supernatants were added to the cells for 6 hours in the presence of polybrene (4 µg/ml) and diluted two times with fresh medium for the next 15 hours.

### Isolation of adipocytes and stromal vascular fraction (SVF) from adipose tissue

Eights week-old male C57BL/6J mice (n=6-8) were euthanized by CO₂ inhalation and epididymal (visceral) and subcutaneous adipose tissue were collected and placed in DMEM medium containing 10mg/mL fatty acid-poor BSA (Sigma-Aldrich, St. Louis, MI). The tissue was minced into fine pieces and then digested in 0.12 units/mL collagenase type I (Sigma) at 37°C in a shaking water bath (80Hz) for 1 hour. Samples were then filtered through a sterile 250µm nylon mesh (Scrynel NY250HC, Milian) to remove undigested fragments. The resulting suspension was centrifuged at 1100 RPM for 10 min to separate SVF from adipocytes. Adipocytes were removed and washed with DMEM buffer. They were then suspended in peqGOLD TriFast reagent (Axonlab) and RNA was isolated according to the manufacturer's instructions. The SVF fraction was incubated in erythrocyte lysis buffer (0.154mM NH₄Cl, 10mM KHCO₃, 0.1mM EDTA) for 2 min. Cells were then centrifuged at 1100 RPM for 10 min and re-suspended in 500 µl of peqGOLD TriFast reagent (Axonlab) for RNA isolation.

### RNA extraction and Real-time PCR

Total RNA was isolated from cultured cells using peqGOLD TriFast reagent according to the manufacturer's instructions (Axonlab). First strand cDNA was synthesized from 0.5 µg of total RNA using random primers and Superscript II (Invitrogen). Real time PCR was performed using Power SYBR Green Mix (Applied Biosystem). The following primers were used for mouse genes: SEQ ID NO.9 (mSt3Gal6-F), SEQ ID NO. 10 (St3Gal6-R), SEQ ID NO. 17 (Ap2-F), SEQ ID NO.18 (Ap2-R), SEQ ID NO. 13 (mCyclophilin A-F), SEQ ID NO. 14 (mCyclophilin A-R). The following primers were used for human genes: SEQ ID NO. 11 (hSt3Gal6-F), SEQ ID NO. 12 (hSt3Gal6-R), SEQ ID NO. 15 (hCyclophilin A-F), SEQ ID NO. 16 (hCyclophilin A-R).

### Northern Blot

Total RNA from various mouse tissues was isolated using the peqGOLD TriFast reagent according to the manufacturer's instructions (Axonlab). Total RNA (8 µg) was separated on a 1.2 % agarose/forlmaldehyde gel and transfected overnight to a nylon membrane. To control for RNA quantity loading, the membrane was stained with methylene blue prior the subsequent hybridizations. For the detection of St3gal6 signals, probes from the full-length cDNA mouse plasmid (Open Biosystem) were used. The probes were labeled by random priming with [α-³²P]dCTP (Amersham). Hybridization and washing were carried out using the Quickhib method according to manufacturer's instructions (Stratagene). Blots were exposed to Hyperfilm ECL (Amersham) at -80°C for 1 day or several days depending on the signal intensity.

### Results

### Example 1: Microarray results

Bioinformatic analysis of the microarray data was performed to identify genes that fulfilled the three following criteria: (i) regulated by high fat feeding, (ii) similar regulated expression by high fat feeding in both visceral (VAT) and subcutaneous fat (SCAT) and (iii) similar normalization of their expression by rimonabant treatment (Figure 1). Out of the ∼17'000 gene targets present on the cDNA microarray used, 34 genes fulfilled these criteria, which are listed in Table 1. Remarkably, 10 of these genes - Cav1, Fgf1, Fndc3b, Kif5b, Mest, Npr3, Pik3ca, Sparc, Vldlr, and Wwtr1 - were previously known to be important regulators of adipose tissue development and function. Some of these genes had expression levels correlated with body weight gain (shown in grey in Table 1), suggesting a potential role in hyperplasia and/or hypertrophy of adipose tissues during obesity. These results validate the approach used to identify possible novel targets for therapeutic treatment of obesity.

Most importantly, many of the genes cited in table 1 have never been studied in the context of in adipose tissue development or biology. These genes belong to the following classes of function: extracellular matrix/cell interaction, cytoskeleton, intracellular signaling, enzymes, and transcription factors/co-factors. They are likely involved in tissue remodeling, and particularly in adipocyte development. One of these genes, St3Gal6 gene and it role in adipocyte biology, is presented herein and constitutes one aspect of the present invention.

The mouse and human sequences of St3Gal6 (NM_018784 and NM_006100 respevtively) as used in the present invention corresponds to SEQ ID NO.1 and SEQ ID NO.3 respectively.

**Table 1: List of 34 gene candidates regulated by HFD and rimonabant in SCAT and VAT. The full name and gene symbol are showed in the first column. The biological role for known genes and references are indicated in the second column. All these genes were up-regulated by HFD and normalized by rimonabant treatment, excepted for Plac8 and Rp9h, which were down-regulated by HFD. The genes correlated to body weight increase are shown in grey.**

| **Gene name** | **Biological function and references** |
|---|---|
| Acetyl-Coenzyme A dehydrogenase, medium chain (Acadm) | |
| ARP2 actin-related protein 2 homolog (Actr2) | |
| Amyloid beta (A4) precursor protein (App) | |
| Annexin A2 (Anxa2) | Role in actin-assembly, |
| Calmodulin 1 (Caim1) | |
| Caveolin, caveolae protein 1 Cav1) | Role in lipid homeostasis, |
| Cyclin G1 (Ccgn1) | |
| Cold shock domain containing E1 (Csde) | |
| Expressed sequence AW112037 | |
| Fibroblast growth factor 1 (Fgf1) | Regulator of human adipogenesis, |
| Fibronectin type III domain containing 3B (Fndc3b) | Role in adipogenesis, |
| Kinesin family member 5B (Kif5b) | Role in insulin-stimulated GLUT4 translocation to the plasma membrane, |
| Mesoderm specific transcript (Mest) | Adipocyte differentiation and enlargement, |
| Nucleosome assembly protein 1-like 1 (Nap1L1) | |
| Nidogen 1 (Nid1) | |
| Natriuretic peptide receptor 3 (Npr3) | Possible role in sodium retention characteristic of obesity associated hypertension, |
| nuclear undecaprenyl pyrophosphate synthase 1 homolog (Nus1) | |
| Phosphatidylinositol 3-kinase, catalytic, alpha polypeptide (Pik3ca) | Essential for proper growth factor signaling. Role in adipogenesis. |
| Placenta-specific 8 (Plac8) | |
| Pleckstrin homology domain containing, family C (Plekhc1) | |
| Protein tyrosine phosphatase 4a1 (Ptp4a1) | Implicated in cell growth, differentiation, and tumor invasion, |
| Related RAS viral (Rras2) oncogene homolog 2 | |
| Retinitis pigmentosa 9 homolog (Rp9h) | |
| Secreted acidic cysteine rich glycoprotein (Sparc) | Mediates cell-matrix interactions and play a differentiation and angiogenesis, |
| Signal-induced proliferation-associated 1 like 1 (Sipa1L1) | |
| Spectrin beta 2 (Spnb2) | |
| **St3 beta-galactoside alpha-2,3-sialyltransferase 6 (St3gal6)** | |
| Vestigial like 3 (VgII3) | |
| Very low density lipoprotein receptor (Vldlr) | Involved in lipolysis, |
| Zinc finger, DHHC domain containing 2 (Zdhhc2) | |
| WD repeat domain 26 (Wdr26) | |
| WW domain containing transcription regulator 1 (Wwtr1) | regulates mesenchymal stem cell differentiation, |
| Expressed sequence AW112037 | |
| RIKEN cDNA B930093H17 gene (like-glycosyltransferase) | |

### Example 2: Tissue and cellular expression of the selected genes

To better understand the role of St3Gal6 in adipocytes development, its pattern of expression was first characterized. mRNA levels were measured by northern-blot and RT-PCR in various mouse tissues, in isolated preadipocytes and adipocytes, in visceral adipose tissue (VAT) and subcutaneous adipose tissue (SCAT) of mouse obesity model (Ob/Ob mice) and in human adipose tissues.

By northern-blotting, it was shown that St3Gal6 is strongly expressed in muscle, heart, lung and kidney, whereas a weaker expression is observed in adipose tissues (BAT, SCAT and VAT) (signals at 2.1 and 1.5 kb indicated by arrows in figure 2A). There is no expression of St3gal6 in liver or spleen (Figure 2A). The expression patterns of St3Gal6 were then observed by RT-PCR studies. In white adipose tissues of Ob/Ob mice St3Gal6level is increased compared to level in wild type mice (Figure 2B). Values are expressed as fold increase relative to the control values in SCAT set arbitrarily at 1.

Adipose tissue is a complex tissue that includes not only mature adipocytes, but also precursor cells such as preadipocytes as well as blood vessels, macrophages and fibroblastic cells. Based on a collagenase I digestion technique, stromal vascular fraction (SVF) (including preadipocyte, endothelial and macrophage cells) was separated from the isolated adipocyte fraction. It was found that St3Gal6 is expressed in both fractions, SVF and isolated adipocytes (Figure 2C). These results indicate that St3Gal6 is involved in differentiationand/or proliferation processes but also immature adipocyte biology.

The next step was to determine whether St3gal6 gene is conserved among species. To address this question, a RT-PCR was performed on human adipose tissue samples. Preadipocytes and adipocytes were isolated from SCAT or VAT. Isolated preadipocytes were induced to differentiate *in vitro* until day 7. Results showed that St3Gal6 is indeed expressed in human fat (Figure 2D).They indicate that these genes are present in human adipose tissues. Altogether these results suggest that St3Gal6 is a relevant candidate gene for adipocytes development, possibly required for adipogenesis or fat tissue enlargement in obesity.

### Example 3: Expression of selected genes during 3T3-L1 differentiation

Next, the expression of St3gal6 gene was assessed during adipogenesis. For that purpose, mRNA levels were measured by RT-PCR during a detailed differentiation time-course of 3T3-L1 (an adipogenic cell line) (Figure 2E). The experiment showed that St3gal6 increased gradually to reach a maximum level after 7 days, suggesting its role in mature adipocyte biology

### Example 4: shRNA knockdown of St3gal6 in 3T3-L1 cells reduces adipogenesis

For the loss-of-function studies, shRNA specific for St3gal6 subcloned into a retroviral vector from Clontech were used (RNAi-Ready pSIREN-RetroQ ZsGreen or pSIREN). This plasmid contains a GFP marker, which allows to control the infection efficiency in 3T3-L1 cells. Three different shRNA for St3gal6, were cloned into the pSIREN plasmid, and were first tested in 293T HEK cells. This experiment demonstrated the ability of shRNA specific for St3gal6 to inhibit St3gal6 expression. Interestingly, 80 % of knockdown was obtained with shSt3gal6-1 (Figure 3A), which and been used for transduction into 3T3-L1 cells.

3T3-L1 cells were then infected for 6 hours with retroviral vectors expressing shRNA directed towards either St3gal6 (sh St3gal6) or luciferase (shLuc). Using the GFP marker, we observed 95 % infection in the 3T3-L1 cells. At day 0, a 60 % knockdown for St3gal6 was obtained in cells infected with shSt3gal6 (Figure 3B) whereas no inhibition was obtained with shLuc control. Then, cells were allowed to reach confluence and after one week differentiated with DMI. After 7 to 10 days of differentiation, cells were stained to determine the amount of lipid content with oil-red-O staining. Knockdown of St3gal6 has no statistically significant effect on adipogenesis *in vitro* (Figure 3C and 3D). In fact, we hypothezised that St3gal6 rather have an effect on the interaction between the developing adipocytes and the extracellular matrix. If so, this effect of St3gal6 knockdown would only be mesurable in a 3-dimentional structure *in vitro* or *in vivo.*

### Bibliography

Baisch G, Ohrlein R, Kolbinger F, Streiff M. 1998. On the preparative use of recombinant pig alpha(1-3)galactosyl-transferase. Bioorganic and medicinal Chemistry letters 8, ppl57-160.
Banerjee, S. S., M. W. Feinberg, M. Watanabe, S. Gray, R. L. Haspel, D. J. Denkinger, R. Kawahara, H. Hauner, and M. K. Jain. 2003. The Kruppel-like factor KLF2 inhibits peroxisome proliferator-activated receptor-gamma expression and adipogenesis. J Biol Chem. 278:2581-4. Epub 2002 Nov 7.
Chen, Z., J. I. Torrens, A. Anand, B. M. Spiegelman, and J. M. Friedman. 2005. Krox20 stimulates adipogenesis via C/EBPbeta-dependent and -independent mechanism. Cell Metab. 2005 Feb;1(2):93-106.
Chun, T. H., K. B. Hotary, F. Sabeh, A. R. Saltiel, E. D. Allen, and S. J. Weiss. 2006. A pericellular collagenase directs the 3-dimensional development of white adipose tissue. Cell 125:577-91.
Gagnon, A. M., J. Chabot, D. Pardasani, and A. Sorisky. 1998. Extracellular matrix induced by TGFbeta impairs insulin signal transduction in 3T3-L1 preadipose cells. J Cell Physiol 175:370-8.
Gray, S., M. W. Feinberg, S. Hull, C. T. Kuo, M. Watanabe, S. Sen-Banerjee, A. DePina, R. Haspel, and M. K. Jain. 2002. The Kruppel-like factor KLF15 regulates the insulin-sensitive glucose transporter GLUT4. J Biol Chem 277:34322-8.
Gregoire, F. M., C. M. Smas, and H. S. Sul. 1998. Understanding adipocyte differentiation. Physiol Rev 78:783-809.
Hausman, G. J., J. T. Wright, and R. L. Richardson. 1996. The influence of extracellular matrix substrata on preadipocyte development in serum-free cultures of stromal-vascular cells. J Anim Sci 74:2117-28.
Jimenez, M. A., P. Akerblad, M. Sigvardsson, and E. D. Rosen. 2007. Critical role for Ebf1 and Ebf2 in the adipogenic transcriptional cascade. Mol Cell Biol 27:743-57.
Kang, S., C. N. Bennett, I. Gerin, L. A. Rapp, K. D. Hankenson, and O. A. Macdougald. 2007. Wnt signaling stimulates osteoblastogenesis of mesenchymal precursors by suppressing CCAAT/enhancer-binding protein alpha and peroxisome proliferator-activated receptor gamma. J Biol Chem 282:14515-24.
Mehlhorn, A. T., P. Niemeyer, S. Kaiser, G. Finkenzeller, G. B. Stark, N. P. Sudkamp, and H. Schmal. 2006. Differential expression pattern of extracellular matrix molecules during chondrogenesis of mesenchymal stem cells from bone marrow and adipose tissue. Tissue Eng 12:2853-62.
Nakajima, I., S. Muroya, R. Tanabe, and K. Chikuni. 2002. Extracellular matrix development during differentiation into adipocytes with a unique increase in type V and VI collagen. Biol Cell 94:197-203.
Okajima, T., S. Fukumoto, H. Miyazaki, H. Ishida, M. Kiso, K. Furukawa, T. Urano, and K. Furukawa. 1999. Molecular cloning of a novel alpha2,3-sialyltransferase (ST3Gal VI) that sialylates type II lactosamine structures on glycoproteins and glycolipids. J Biol Chem 274:11479-86.
Oyama T, Miyoshi Y, Koyama K, Nakagawa H, Yamori T, Ito T, Matsuda H, Arakawa H, Nakamura Y.2000. Isolation of a novel gene on 8p21.3-22 whose expression is reduced significantly in human colorectal cancers with liver metastasis. Genes Chromosomes Cancer. Sep;29(1):9-15.
Rosen ED, MacDougald OA. 2006. Adipocyte differentiation from the inside out. Nat Rev Mol Cell Biol. 2006 Dec;7(12):885-96.
Rosen ED, Spiegelman BM.Adipocytes as regulators of energy balance and glucose homeostasis. Nature. 2006 Dec 14;444(7121 ):847-53.
Rosen, E. D., C. H. Hsu, X. Wang, S. Sakai, M. W. Freeman, F. J. Gonzalez, and B. M. Spiegelman. 2002. C/EBPalpha induces adipogenesis through PPARgamma: a unified pathway. Genes Dev 16:22-6.
Sewell R, Bäckström M, Dalziel M, Gschmeissner S, Karlsson H, Noll T, Gätgens J, Clausen H, Hansson GC, Burchell J, Taylor-Papadimitriou J. 2006. J Biol Chem. The ST6GalNAc-I sialyltransferase localizes throughout the Golgi and is responsible for the synthesis of the tumor-associated sialyl-Tn O-glycan in human breast cancer. 281 (6):3586-94.

## Claims

1. Use of an inhibitor of the activity St3Gal6 for the modulation of adipogenesis.

2. Use according to claim 1 wherein said modulator is used to reduce adipogenesis.

3. Use according to claim 1 or 2 for the treatment of obesity and related disorders.

4. Use according to claim 1 or 2 for the reduction of visceral and/or subcutaneous fat accumulation.

5. Use according to any previous claim wherein said inhibitor is a small molecule.

6. Use according to any previous claim wherein said inhibitor is small interfering RNA.

7. Use according to claim 6 wherein the siRNA is a shRNA having a sequence corresponding to SEQ ID NO. 5.

8. Nucleic acid being a siRNA specific for St3Gal6 transcriptional inhibition.

9. Nucleic acid having sequence SEQ ID NO. 5.

10. Method for identification of a compound able to modulate the enzymatic activity of St3Gal6 wherein the activity of sialylation is measured in a sample containing St3Gal6 activity after addition of a candidat compound and compared with the activity of sialylation present in a control sample and wherein a candidat compound is identified when a decrease in the sialylation activity is measured compared to the activity measured in a control.

11. Composition comprising an inhibitor of St3Gal6 activity and at least one pharmaceutically acceptable excipient.

12. Composition according to claim 11 for the preparation of a medicament to inhibit adipogenesis.

13. Composition according to claim 12 to treat obesity and related diseases.

14. Composition according to claim 11 for reduction of visceral and/or subcutaneous fat accumulation.

15. Method of modulation of adipogenesis consisting in administration to a patient in need thereof of an inhibitor of St3Gal6 to modulate adipogenesis.


SEQUENCE LISTING

<110>  SANOFI-AVENTIS

<120>  Use of inhibitors of St3Gla6 activity for modulation of
       adpogenesis

<130>  FR2009-027

<160>  18

<170>  PatentIn version 3.3

<210>  1
<211>  1593
<212>  DNA
<213>  Mus musculus


<220>
<221>  CDS
<222>  (498)..(1487)

<400>  1
ggggaacgtt ggcggtctca gcctccgggc cacactgcgc ctcgctccgc tctgctcctt      60

cgccacatcg ggggtagggg tggagggaag ggatcgcgtt cttgtcaccc cgctagctca      120

ggcttccgct cgccgcctgc tcgggacccg atgctgcaga cgccacggga cagacagaag      180

cgcgctgcgg agagccctgt gcatgcctct gcggtggccg agctcgggcc tcggcagcac      240

gcacacgtga agcaacaaca aagcccctca tcttggatta tttccagcat caagacctcc      300

acatgtcaac agcaggctcc tgaggcagtc catcctcctc caactctcct cactacattc      360

ttcagaaaga gagggtcttc aggaaacaca ccccaaaagc gcagatttat ttactaattc      420

atgtatttga accatcgtgg aagacctctg catattcatc tcccctcact ttggactctg      480

tttcaggcag gccagcc atg aaa ggg tat ctg gtg gcc ata ttc ctg agt      530
                   Met Lys Gly Tyr Leu Val Ala Ile Phe Leu Ser
                   1               5                   10

tcc atc ttc ctc tat tat gta cta tac tgt ata ctg tgg gga aca aat      578
Ser Ile Phe Leu Tyr Tyr Val Leu Tyr Cys Ile Leu Trp Gly Thr Asn
            15                  20                  25

ggc tat tgg ttc cca gct gaa gaa atg agg act aga aac aat gtc aat      626
Gly Tyr Trp Phe Pro Ala Glu Glu Met Arg Thr Arg Asn Asn Val Asn
        30                  35                  40

aat tgt ttt aaa aag cca gct ttc gcc aat ctt ctg aga ttt cct cag      674
Asn Cys Phe Lys Lys Pro Ala Phe Ala Asn Leu Leu Arg Phe Pro Gln
    45                  50                  55

ctt tac cca ttt ctg tgc aga gct gac ttt ata aag gtt gct gcc atg      722
Leu Tyr Pro Phe Leu Cys Arg Ala Asp Phe Ile Lys Val Ala Ala Met
60                  65                  70                  75

tcc ggt acc aat aat ttt ccg ttg ccc tat gga ata aag acc ttc gag      770
Ser Gly Thr Asn Asn Phe Pro Leu Pro Tyr Gly Ile Lys Thr Phe Glu
                80                  85                  90

aca tat ttc agc tcg gcc ctt tca aaa ctg cag agt tgt gat ctc ttt      818
Thr Tyr Phe Ser Ser Ala Leu Ser Lys Leu Gln Ser Cys Asp Leu Phe
            95                  100                 105

gac gag ttt gac aga gtg cca tgt aaa agg tgt gtg gtg gtt ggt aat 866
Asp Glu Phe Asp Arg Val Pro Cys Lys Arg Cys Val Val Val Gly Asn
        110                 115                 120

gga gga gtg ttg aag aat aag aca tta gga gca aca att gac tcc tat 914
Gly Gly Val Leu Lys Asn Lys Thr Leu Gly Ala Thr Ile Asp Ser Tyr
    125                 130                 135

gat gta ata ata aga atg aac aac ggt cct gtc tta ggc cat gaa gag 962
Asp Val Ile Ile Arg Met Asn Asn Gly Pro Val Leu Gly His Glu Glu
140                 145                 150                 155

gaa gtt ggg aca aga aca acc ttc agg ctt ttt tat cca gag tct gtc 1010
Glu Val Gly Thr Arg Thr Thr Phe Arg Leu Phe Tyr Pro Glu Ser Val
                160                 165                 170

ttt tca gac tcc agt cac tat gac ccc aat act aca gcg gtt ctc gtc 1058
Phe Ser Asp Ser Ser His Tyr Asp Pro Asn Thr Thr Ala Val Leu Val
            175                 180                 185

gtc ttt aag cca cag gat tta agg tgg ctg gtg gaa ata ctg cta ggt 1106
Val Phe Lys Pro Gln Asp Leu Arg Trp Leu Val Glu Ile Leu Leu Gly
        190                 195                 200

aaa aaa ata aat act caa ggg ttt tgg aag aca cca gcc tta aaa ctg 1154
Lys Lys Ile Asn Thr Gln Gly Phe Trp Lys Thr Pro Ala Leu Lys Leu
    205                 210                 215

atc tat aaa caa tac caa atc aga ata tta gat cca tat atc acc agc 1202
Ile Tyr Lys Gln Tyr Gln Ile Arg Ile Leu Asp Pro Tyr Ile Thr Ser
220                 225                 230                 235

gaa gca gct ttt caa atg ctt cgt ttt ccc aga gta ttt ccc aag gat 1250
Glu Ala Ala Phe Gln Met Leu Arg Phe Pro Arg Val Phe Pro Lys Asp
                240                 245                 250

cag aaa ccc aaa cac cct aca aca gga att att gcc atc aca atg gcc 1298
Gln Lys Pro Lys His Pro Thr Thr Gly Ile Ile Ala Ile Thr Met Ala
            255                 260                 265

ttt cac ata tgc agt gaa gtg cac ctc gct ggt ttt aag tac aac ttt 1346
Phe His Ile Cys Ser Glu Val His Leu Ala Gly Phe Lys Tyr Asn Phe
        270                 275                 280

tac agc ccc aac agt cct tta cac tac tac ggg aat gcc acc atg tct 1394
Tyr Ser Pro Asn Ser Pro Leu His Tyr Tyr Gly Asn Ala Thr Met Ser
    285                 290                 295

ttg atg aag cag aat gca tat cac aat ctg act gca gag cag ctc ttt 1442
Leu Met Lys Gln Asn Ala Tyr His Asn Leu Thr Ala Glu Gln Leu Phe
300                 305                 310                 315

tta aac gac att ata aag aaa aaa atg gtg atc aac ttg act taa 1487
Leu Asn Asp Ile Ile Lys Lys Lys Met Val Ile Asn Leu Thr
                320                 325

aattgaccct atggatccaa aagatgatga tgctaaacag tattagtttt atttttgtac 1547

tgcaaatttt agtttatttt taaatatatt ggatgaactt atcaaa 1593

<210> 2
<211> 329
<212> PRT
<213> Mus musculus

<400> 2

Met Lys Gly Tyr Leu Val Ala Ile Phe Leu Ser Ser Ile Phe Leu Tyr
1               5                   10                  15

Tyr Val Leu Tyr Cys Ile Leu Trp Gly Thr Asn Gly Tyr Trp Phe Pro
            20                  25                  30

Ala Glu Glu Met Arg Thr Arg Asn Asn Val Asn Asn Cys Phe Lys Lys
        35                  40                  45

Pro Ala Phe Ala Asn Leu Leu Arg Phe Pro Gln Leu Tyr Pro Phe Leu
    50                  55                  60

Cys Arg Ala Asp Phe Ile Lys Val Ala Ala Met Ser Gly Thr Asn Asn
65                  70                  75                  80

Phe Pro Leu Pro Tyr Gly Ile Lys Thr Phe Glu Thr Tyr Phe Ser Ser
                85                  90                  95

Ala Leu Ser Lys Leu Gln Ser Cys Asp Leu Phe Asp Glu Phe Asp Arg
            100                 105                 110

Val Pro Cys Lys Arg Cys Val Val Val Gly Asn Gly Gly Val Leu Lys
        115                 120                 125

Asn Lys Thr Leu Gly Ala Thr Ile Asp Ser Tyr Asp Val Ile Ile Arg
    130                 135                 140

Met Asn Asn Gly Pro Val Leu Gly His Glu Glu Glu Val Gly Thr Arg
145                 150                 155                 160

Thr Thr Phe Arg Leu Phe Tyr Pro Glu Ser Val Phe Ser Asp Ser Ser
                165                 170                 175

His Tyr Asp Pro Asn Thr Thr Ala Val Leu Val Val Phe Lys Pro Gln
            180                 185                 190

Asp Leu Arg Trp Leu Val Glu Ile Leu Leu Gly Lys Lys Ile Asn Thr
        195                 200                 205

Gln Gly Phe Trp Lys Thr Pro Ala Leu Lys Leu Ile Tyr Lys Gln Tyr
    210                 215                 220

Gln Ile Arg Ile Leu Asp Pro Tyr Ile Thr Ser Glu Ala Ala Phe Gln
225                 230                 235                 240

Met Leu Arg Phe Pro Arg Val Phe Pro Lys Asp Gln Lys Pro Lys His
                245                 250                 255

Pro Thr Thr Gly Ile Ile Ala Ile Thr Met Ala Phe His Ile Cys Ser
            260                 265                 270

Glu Val His Leu Ala Gly Phe Lys Tyr Asn Phe Tyr Ser Pro Asn Ser
        275                 280                 285

Pro Leu His Tyr Tyr Gly Asn Ala Thr Met Ser Leu Met Lys Gln Asn
    290                 295                 300

Ala Tyr His Asn Leu Thr Ala Glu Gln Leu Phe Leu Asn Asp Ile Ile
305                 310                 315                 320

Lys Lys Lys Met Val Ile Asn Leu Thr
                325

<210>  3
<211>  2107
<212>  DNA
<213>  Homo sapiens

<220>
<221>  CDS
<222>  (468)..(1463)

<400>  3
gagcctgaga ctccgggcag ggctgctccc tcctctgctc ccccgccaga tccgcgggga      60

aggaatcgtg cccgcgccgc ccctggcccg cgccaccttc ctttggtttc tgccggcctc     120

gggcttctgc ggcccgatgt ggcaggcgcc gcgagagagg cagcagccgg ctggagcagc     180

ggcccctcag gtctcggagc ccggtgcgcc tctgcggtcg tcgctcctgg gcctcggcgg     240

gtcactcttg ccggccggct tcgctgcggg tttgcactgc ccggcatttg caagtgagga     300

agcagcacaa ccctgggttt tagataattt ctaacagaga ggccccagca attcagcagg     360

cagcgtcctg agtctttggc agcctggtcc ctctttcgca aattctccac ctttgcgaac     420

agagggtctt taggacacag attcttaaaa gtgcaggtga gccagcc atg aga ggg      476
                                                    Met Arg Gly
                                                    1

tat ctt gtg gcc ata ttc ctg agt gct gtc ttc ctc tat tat gta ctg      524
Tyr Leu Val Ala Ile Phe Leu Ser Ala Val Phe Leu Tyr Tyr Val Leu
    5                   10                  15

cat tgc ata tta tgg gga acg aat gtc tat tgg gtg gca cct gtg gaa      572
His Cys Ile Leu Trp Gly Thr Asn Val Tyr Trp Val Ala Pro Val Glu
20                  25                  30                  35

atg aaa cgg aga aat aag atc cag cct tgt tta tca aag cca gct ttt      620
Met Lys Arg Arg Asn Lys Ile Gln Pro Cys Leu Ser Lys Pro Ala Phe
                40                  45                  50

gcc tct ctg ctg agg ttt cat cag ttt cac cct ttt ctg tgt gcg gct      668
Ala Ser Leu Leu Arg Phe His Gln Phe His Pro Phe Leu Cys Ala Ala
            55                  60                  65

gat ttt aga aag att gct tcc ttg tat ggt agc gat aag ttt gat ttg      716
Asp Phe Arg Lys Ile Ala Ser Leu Tyr Gly Ser Asp Lys Phe Asp Leu
        70                  75                  80

ccc tat ggg atg aga aca tca gcg gaa tat ttt cga ctt gct ctt tca      764
Pro Tyr Gly Met Arg Thr Ser Ala Glu Tyr Phe Arg Leu Ala Leu Ser
    85                  90                  95

aaa ctg cag agt tgt gat ctc ttt gat gag ttt gac aac ata ccc tgt 812
Lys Leu Gln Ser Cys Asp Leu Phe Asp Glu Phe Asp Asn Ile Pro Cys
100                 105                 110                 115

aaa aag tgt gtg gtg gtt ggt aat gga gga gtt ttg aag aat aag aca 860
Lys Lys Cys Val Val Val Gly Asn Gly Gly Val Leu Lys Asn Lys Thr
                120                 125                 130

tta gga gaa aaa atc gac tcc tat gat gta ata ata aga atg aat aat 908
Leu Gly Glu Lys Ile Asp Ser Tyr Asp Val Ile Ile Arg Met Asn Asn
            135                 140                 145

ggt cct gtt tta gga cat gaa gaa gaa gtt ggg aga agg aca acc ttc 956
Gly Pro Val Leu Gly His Glu Glu Glu Val Gly Arg Arg Thr Thr Phe
        150                 155                 160

cga ctt ttt tat cca gaa tct gtt ttt tca gat cct att cac aat gac 1004
Arg Leu Phe Tyr Pro Glu Ser Val Phe Ser Asp Pro Ile His Asn Asp
    165                 170                 175

cct aat acg aca gtg att ctc act gct ttt aag cca cat gat tta agg 1052
Pro Asn Thr Thr Val Ile Leu Thr Ala Phe Lys Pro His Asp Leu Arg
180                 185                 190                 195

tgg ctg ttg gaa ttg ttg atg ggt gac aaa ata aac act aat ggt ttt 1100
Trp Leu Leu Glu Leu Leu Met Gly Asp Lys Ile Asn Thr Asn Gly Phe
                200                 205                 210

tgg aag aaa cca gcc tta aac ctg att tat aaa cct tat caa atc cga 1148
Trp Lys Lys Pro Ala Leu Asn Leu Ile Tyr Lys Pro Tyr Gln Ile Arg
            215                 220                 225

ata tta gat cct ttc att atc aga aca gca gct tat gaa ctg ctt cat 1196
Ile Leu Asp Pro Phe Ile Ile Arg Thr Ala Ala Tyr Glu Leu Leu His
        230                 235                 240

ttt cca aaa gtg ttt ccc aaa aat cag aaa cct aaa cac cca aca aca 1244
Phe Pro Lys Val Phe Pro Lys Asn Gln Lys Pro Lys His Pro Thr Thr
    245                 250                 255

gga att att gcc atc aca ttg gcg ttt tac ata tgt cac gaa gtt cac 1292
Gly Ile Ile Ala Ile Thr Leu Ala Phe Tyr Ile Cys His Glu Val His
260                 265                 270                 275

cta gct ggt ttt aaa tac aac ttt tct gac ctc aag agt cct ttg cac 1340
Leu Ala Gly Phe Lys Tyr Asn Phe Ser Asp Leu Lys Ser Pro Leu His
                280                 285                 290

tac tat ggg aat gcc acc atg tct ttg atg aat aag aac gcg tat cac 1388
Tyr Tyr Gly Asn Ala Thr Met Ser Leu Met Asn Lys Asn Ala Tyr His
            295                 300                 305

aat gtg act gca gag cag ctc ttt ttg aag gac att ata gaa aaa aac 1436
Asn Val Thr Ala Glu Gln Leu Phe Leu Lys Asp Ile Ile Glu Lys Asn
        310                 315                 320

ctc gta atc aac ttg act caa gat tga ctctacagac tcagaagatg 1483
Leu Val Ile Asn Leu Thr Gln Asp
    325                 330

atgctaacag tgttagtttt atttttgtac tgcaattttt agtttaaaat atgttggatg 1543

cactcgtcaa ataattatgt atactgtctg ttgctgcctg gtgattcata accaccagct 1603

taatttctgt gaatactgta tatttaactt atgaaaacca agaaatgtaa agataacagg 1663

aaaataagtt ttgattgcaa tgtttttaaa ataagctagt tttctgaggt gttttcacac 1723

gtctttttat agttacttca tcttagattt ttgaagggat atgacttcct actaaggatt 1783

tagtttacca caacaattct gactacaata agacattttg aggaggatat ttggctactg 1843

taaacatggc tggtggaaaa tcacgattgt ggcttgatgt ggcaagccga aaccacttgg 1903

ctctggaaat ctaagttcat actggtttaa ttaagctctc tcctgacaac ccccagaatt 1963

aaatgaacca tgattgtgaa gagtaatttg gtacaatgaa ggcagtgttt gtttttaagt 2023

taaaggaaat gggctaaaca taaagttctt attagataag taaataacta aagaaagaat 2083

acaattacta aaaaaaaaaa aaaa 2107

<210> 4
<211> 331
<212> PRT
<213> Homo sapiens

<400> 4

Met Arg Gly Tyr Leu Val Ala Ile Phe Leu Ser Ala Val Phe Leu Tyr
1               5                   10                  15

Tyr Val Leu His Cys Ile Leu Trp Gly Thr Asn Val Tyr Trp Val Ala
            20                  25                  30

Pro Val Glu Met Lys Arg Arg Asn Lys Ile Gln Pro Cys Leu Ser Lys
        35                  40                  45

Pro Ala Phe Ala Ser Leu Leu Arg Phe His Gln Phe His Pro Phe Leu
    50                  55                  60

Cys Ala Ala Asp Phe Arg Lys Ile Ala Ser Leu Tyr Gly Ser Asp Lys
65                  70                  75                  80

Phe Asp Leu Pro Tyr Gly Met Arg Thr Ser Ala Glu Tyr Phe Arg Leu
                85                  90                  95

Ala Leu Ser Lys Leu Gln Ser Cys Asp Leu Phe Asp Glu Phe Asp Asn
            100                 105                 110

Ile Pro Cys Lys Lys Cys Val Val Val Gly Asn Gly Gly Val Leu Lys
        115                 120                 125

Asn Lys Thr Leu Gly Glu Lys Ile Asp Ser Tyr Asp Val Ile Ile Arg
    130                 135                 140

Met Asn Asn Gly Pro Val Leu Gly His Glu Glu Glu Val Gly Arg Arg
145                 150                 155                 160

Thr Thr Phe Arg Leu Phe Tyr Pro Glu Ser Val Phe Ser Asp Pro Ile
                165                 170                 175

His Asn Asp Pro Asn Thr Thr Val Ile Leu Thr Ala Phe Lys Pro His

            180                 185                 190

Asp Leu Arg Trp Leu Leu Glu Leu Leu Met Gly Asp Lys Ile Asn Thr
        195                 200                 205

Asn Gly Phe Trp Lys Lys Pro Ala Leu Asn Leu Ile Tyr Lys Pro Tyr
    210                 215                 220

Gln Ile Arg Ile Leu Asp Pro Phe Ile Ile Arg Thr Ala Ala Tyr Glu
225                 230                 235                 240

Leu Leu His Phe Pro Lys Val Phe Pro Lys Asn Gln Lys Pro Lys His
                245                 250                 255

Pro Thr Thr Gly Ile Ile Ala Ile Thr Leu Ala Phe Tyr Ile Cys His
            260                 265                 270

Glu Val His Leu Ala Gly Phe Lys Tyr Asn Phe Ser Asp Leu Lys Ser
        275                 280                 285

Pro Leu His Tyr Tyr Gly Asn Ala Thr Met Ser Leu Met Asn Lys Asn
    290                 295                 300

Ala Tyr His Asn Val Thr Ala Glu Gln Leu Phe Leu Lys Asp Ile Ile
305                 310                 315                 320

Glu Lys Asn Leu Val Ile Asn Leu Thr Gln Asp
                325                 330

<210> 5
<211> 65
<212> DNA
<213> Artificial

<220>
<223> scRNA

<220>
<221> misc_RNA
<222> (1)..(65)
<223> shSt3gal6-1

<400> 5
gatccgaaca acggtcctgt cttattcaag agataagaca ggaccgttgt tcttttttac 60

gcgtg 65

<210> 6
<211> 65
<212> DNA
<213> Artificial

<220>
<223> scRNA

<220>
<221>  misc_RNA
<222>  (1)..(65)
<223>  shSt3gal6-2

<400>  6
gatccgtgga agacctctgc atatttcaag agaatatgca gaggtcttcc acttttttac      60

gcgtg                                                                  65


<210>  7
<211>  66
<212>  DNA
<213>  Artificial

<220>
<223>  scRNA


<220>
<221>  misc_RNA
<222>  (1)..(66)
<223>  shSt3gal6-3

<400>  7
gatccgccaa tactacagcg gttctttcaa gagaagaacc gctgtagtat tggtttttta      60

cgcgtg                                                                 66


<210>  8
<211>  63
<212>  DNA
<213>  Artificial

<220>
<223>  scRNA


<220>
<221>  misc_RNA
<222>  (1)..(63)
<223>  shLuc

<400>  8
gatccgtgcg ttgctagtac caattcaaga gattggtact agcaacgcac tttttacgc      60

gtg                                                                    63


<210>  9
<211>  23
<212>  DNA
<213>  Artificial

<220>
<223>  Primer mSt3gal6-F

<400>  9
agcccctcat cttggattat ttc                                              23


<210>  10
<211>  20
<212>  DNA
<213>  Artificial

<220>
<223>  Primer mSt3gal6-R

<400>  10
tcaggagcct gctgttgaca                                                    20


<210>  11
<211>  15
<212>  DNA
<213>  Artificial

<220>
<223>  Primer hSt3gal6-F

<400>  11
cgctgcgggt ttgca                                                         15


<210>  12
<211>  21
<212>  DNA
<213>  Artificial

<220>
<223>  Primer hSt3gal6-R

<400>  12
ggttgtgctg cttcctcact t                                                  21


<210>  13
<211>  20
<212>  DNA
<213>  Artificial

<220>
<223>  Primer mCyclophilin A-F

<400>  13
ttttgacttg cgggcatttt                                                    20


<210>  14
<211>  22
<212>  DNA
<213>  Artificial

<220>
<223>  Primer mCyclophiilin A-R

<400>  14
ggacgctctc ctgagctaca ga                                                 22


<210>  15
<211>  20
<212>  DNA
<213>  Artificial

<220>
<223>  Primer hCyclophiilin A-F

<400>  15
ttcatctgca ctgccaagac                                                    20


<210>  16

<211>  20
<212>  DNA
<213>  Artificial

<220>
<223>  Primer hCyclophiilin A-R

<400>  16
tcgagttgtc cacagtcagc                                                    20


<210>  17
<211>  19
<212>  DNA
<213>  Artificial

<220>
<223>  Primer Ap2-F

<400>  17
ccgcagacga caggaaggt                                                     19


<210>  18
<211>  15
<212>  DNA
<213>  Artificial

<220>
<223>  Primer Ap2-R

<400>  18
agggccccgc catct                                                         15